(19) Europäisches Patentamt
European Patent Office
Office européen des brevets

(11) Publication number: **0 241 837 B1**

# (12) EUROPEAN PATENT SPECIFICATION

(45) Date of publication of patent specification: **27.12.91**

(51) Int. Cl.⁵: **A61K 31/165**, A61K 47/00, A61K 9/08, A61K 47/10, A61K 47/36

(21) Application number: **87105073.8**

(22) Date of filing: **06.04.87**

## (54) Stable injectable antiemetic compositions.

(30) Priority: **07.04.86 US 848615**
**10.03.87 US 19733**

(43) Date of publication of application:
**21.10.87 Bulletin 87/43**

(45) Publication of the grant of the patent:
**27.12.91 Bulletin 91/52**

(84) Designated Contracting States:
**AT BE CH DE ES FR GB GR IT LI LU NL SE**

(56) References cited:
**EP-A- 0 008 525**
**AU-A- 539 625**
**GB-A- 2 062 467**
**GB-A- 2 160 871**
**WO-A-86/02553**
**FR-A- 2 396 550**
**GB-A- 2 158 714**
**US-A- 4 328 213**

(73) Proprietor: **Bristol-Myers Squibb Company**
**345 Park Avenue**
**New York, N.Y. 10154(US)**

(72) Inventor: **Nassar, Munir N.**
**5100 Highbridge Street Nr. 51F**
**Fayetteville, N.Y. 13066(US)**
Inventor: **Agharkar, Shreeram N.**
**7290 Wakefield Drive**
**Fayetteville, N.Y. 13066(US)**
Inventor: **Bogardus, Joseph B.**
**8239 Penstock Way**
**Manlius, New York 13104(US)**

(74) Representative: **Kinzebach, Werner, Dr.**
**Patentanwälte Reitstötter, Kinzebach und Partner Sternwartstrasse 4 Postfach 86 06 49**
**W-8000 München 86(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to stable, injectable, aqueous solutions of 4-amino-5-chloro-N-[2-(diethylamino)-ethyl]-2-[(substituted)alkoxy]benzamide antiemetic agents containing, as stabilizing agent, a pharmaceutically acceptable, water-miscible, hydroxylic organio solvent or water-soluble polyhydric alcohol or sugar which decreases the polarity of the solution.

Methods for the stabilization of medicaments vary widely, depending on the type of medicament. The addition of a reducing agent to an easily oxidized medicament is a well-known example. Ascorbic acid has been stabilized against oxidation by the use of a mixed solvent having a decreased dissolved oxygen content. The degradation (by hydrolysis) of some anaesthetic esters has been inhibited by the addition of caffeine, which was shown to complex with the esters. The series of "paraben" preservatives, e.g. methyl paraben, ethyl paraben, propyl paraben and butyl paraben, are well-known stabilizers used in numerous pharmaceutical compositions. U.S. Patent 4,328,213, issued on May 4, 1982 to V. Ecker et al., for example, describes and claims their use in the stabilization of injectable labetalol formulations.

The antiemetic agents utilized in the stable formulations described and claimed herein are known compounds, having been described, for example, in published U.K. Patent Application 2,160,871 A, published on January 2, 1986.

Metoclopramide is a well-known antiemetic agent with a structure similar to the antiemetic agents utilized herein, except that it contains a methoxy group in the 2-position. The Physicians' Desk Reference, 36th edition, 1982, pages 1565-6, shows that the injectable form of metoclopramide sold at that time was stabilized with sodium metabisulfite.

Published U.K. Patent Application 2,158,714 A, published on November 20, 1985, confirms that injectable metoclopramide formulations were then stabilized with sodium metabisulfite. It goes on to point out that metoclopramide was being used in conjunction with cisplatinum chemotherapy for cancer, and that cisplatinum had been found to be incompatible with sodium metabisulfite. It states that it was found that, surprisingly, sodium metabisulfite could be eliminated from injectable metoclopramide formulations without unduly affecting their stability.

In Chem. Pharm. Bull., 8, 504 (1960), K. Ikeda reports the results of a study which showed increased stability of certain barbiturates in water-ethanol or water-methanol solutions having reduced dielectric constants, as compared with aqueous solutions of the barbiturates.

In Chem. Pharm. Bull., 8, (1960), K. Ikeda reports that the same barbiturates have increased stability in aqueous solutions of ethylene glycol, propylene glycol, glycerol, glucose, mannitol and sucrose. However, his studies showed that the stabilization could not be attributed only to the change of dielectric constant of the medium. Moreover, he refers to the work of E.S. Amis et al. in J. Am. Chem. Soc., 63, 2621 (1940) with the alkaline degradation of bromthymol blue. Amis et al. found that the reaction between negative bivalent dye ion and hydroxyl ion was in accordance with the theory in methanol-water and ethanol-water, but in glycerol-water mixture the activation energy was opposite to the theory on dielectric constant.

In J. Am. Pharm. Assoc., 48, 77 (1959), A.D. Marcus et al. refer to "the widespread use of mixed solvents in pharmaceuticals and the relative lack of information concerning the effects of such solvent systems upon the stability of the active ingredients". They point out that any assumption that replacement of part of the water by a non-aqueous solvent is some sort of a panacea is erroneous, and probably results from a lack of appreciation of the ability of non-aqueous solvents, especially those which are hydroxylic, to participate in, or otherwise influence, solvolytic reactions. In a study of the hydrogen ion catalyzed solvolysis of chloramphenicol in water-propylene glycol solutions, they found that the addition of propylene glycol to the aqueous solution increased the rate of solvolysis of the chloramphenicol.

This invention relates to stable, injectable, aqueous solutions of antiemetic agents having the Formula I

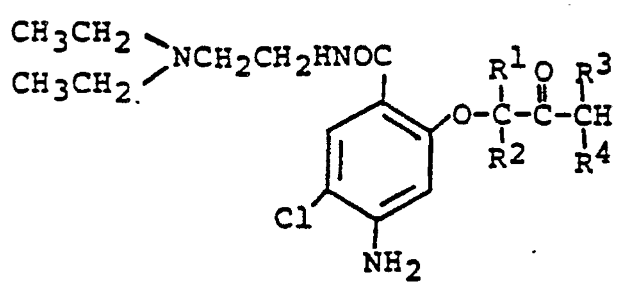

I

wherein $R^1$, $R^2$ and $R^3$ each are independently hydrogen or methyl, and $R^4$ is hydrogen, a straight or branched chain alkyl group containing from 1 to 4 carbon atoms or, when $R^3$ is hydrogen, $R^4$ may be phenyl, or a nontoxic, pharmaceutically acceptable acid addition salt thereof, containing as stabilizing agent a pharmaceutically acceptable, water-miscible, hydroxylic organic solvent or water-soluble polyhydric alcohol or sugar which decreases the polarity (dielectric constant) of the solution. Preferred organic hydroxylic stabilizers include ethanol, propylene glycol glycerol and mannitol, with glycerol being the most preferred. A particularly preferred pharmaceutically acceptable acid addition salt of a compound of Formula I is the hydrochloride. The most preferred compound of Formula I is the compound wherein $R^1$, $R^3$ and $R^4$ are hydrogen, and $R^2$ is methyl, which is named 4-amino-2-(2-butanon-3-yl)-oxy-5-chloro-N-[2-(diethylamino)ethyl]benzamide (Ia).

The compounds of Formula I in aqueous, isotonic (isotonicity adjusted with sodium chloride and/or dextrose) and buffered (0.IM citrate and phosphate buffers, pH 5.7 and 6.5) formulations containing the equivalent of 5 mg of free base of a compound of Formula I per ml have been found unsuitable for long term shelf life. Typical isotonic and buffered formulations lost 33% and 40%, respectively, of their potency following storage for eight weeks at 56° C. The compound of Formula Ia, for example, has been found to degrade via an intramolecular cyclization reaction leading to the formation of the compound of Formula II

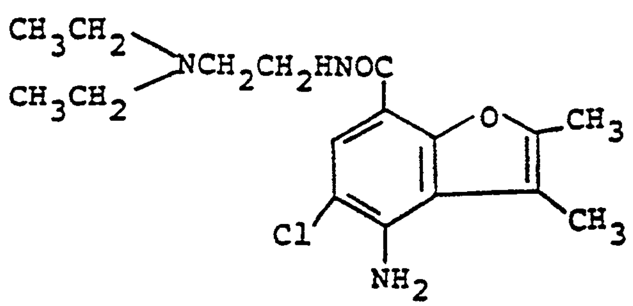

II

which was isolated and identified. Tables 1 and 2 give the results of stability tests on the compound of Formula Ia in water and in isotonic saline, and in citrate buffers, respectively.

Table 1

| Stability of Compound Ia (5mg/ml) in Water and Isotonic Saline at 56° C | | |
|---|---|---|
| Time (Weeks) | % Remaining(a) (Water) | % Remaining(a) (Isotonic Saline) |
| Initial | 100.0 | 100.0 |
| 1 | 97.4 | 96.0 |
| 2 | 95.5 | 91.6 |
| 4 | 90.8 | 83.5 |
| 8 | 80.5 | 66.9 |

(a) Reported values are the average of duplicate samples.

Table 2

| Stability of Compound Ia (5 mg/ml) in 0.01M Citrate Buffers at pH 5.7 and 6.5 at 56° C | | |
|---|---|---|
| Time (Weeks) | % Remaining(a) (pH 5.7) | % Remaining(a) (pH 6.5) |
| Initial | 100.0 | 100.0 |
| 1 | 94.8 | 93.6 |
| 2 | 87.8 | 87.9 |
| 4 | 77.2 | 77.5 |
| 8 | 58.9 | 60.3 |

(a) Reported values are the average of duplicate samples.

According to the present invention it has been found that a stable, injectable, aqueous formulation of a compound of Formula I can be attained by the addition of a pharmaceutically acceptable hydroxylic organic solvent or water soluble polyhydric alcohol or sugar to lower the dielectric constant (polarity) of the solution. Preferred hydroxylic stabilizers include ethanol, propylene glycol, glycerol and mannitol with glycerol being the most preferred.

The amount of hydroxylic stabilizer to be added may vary from 5% up to 75% depending on the actual compound used. However, we prefer to use from 5% to 30%, and most preferably from 10% to 20%. For example, we have found that compositions containing 75% ethanol are among the most stable but, because of potential undesirable side effects from the intravenous administration of moderate amounts of ethanol, we prefer to use much lower amounts of ethanol or even to use a different hydroxylic solvent such as glycerol. Even glycerol, in high dosage may cause undesirable side effects and, for that reason, we most prefer to use the stabilizer at a concentration of 10%.

The amount of Compound I per ml of stable formulation may vary from 1 mg to 50 mg or even more, depending on the particular compound. We prefer to use at least 1 mg/ml and up to 40 mg/ml.

The pH of the final composition should be in the range of 4 to 7, and preferably is from 5.0 to 6.7. Most preferably, the pH is within the range of 6.0 to 6.5.

Optionally, 9 mg/ml of the preservative benzyl alcohol may be added to the formulation. This is particularly desirable if the formulation is packaged in multiple dose form.

The pharmaceutically acceptable acid addition salts of the compounds of Formula I may be derived from any of the acids commonly used in the pharmaceutical art. Preferred salts are the sulfate, maleate, tartrate, citrate and hydrochloride, with the hydrochloride being the most preferred.

The hydroxylic stabilizing compounds utilized herein, such as ethanol, propylene glycol, glycerol and mannitol, all lower the dielectric constant of the formulations. Although we believe that the stabilizing effect is due, at least partially, to the lowered dielectric constant of the formulations, we do not intend to limit ourselves to any particular theory.

The dosage of the compounds of Formula I depend on the particular active ingredient, the age, weight and general health of the patient, as well as the severity of the malady, and is within the discretion of the physician. For the prevention of nausea and vomiting associated with emetogenic cancer chemotherapeutic agents, the compounds of Formula I are generally administered at a dosage of 1 mg/kg to 50 mg/kg, given several times per day.

| Example 1 | |
|---|---|
| Ingredient | Amount/ml |
| Compound Ia Hydrochloride | 5.51 mg |
| Glycerol | 200 mg |
| Sodium Hydroxide (0.01N) | 65 $\mu$1 |
| Water for Injection q.s. ad | 1.00 ml |

Procedure for Formulation

Into a suitable compounding vessel add an excess of the batch volume of Water for Injection. Heat to boiling and allow the water to boil for ten minutes. While cooling, cover the container, bubble nitrogen

through the water and keep a jet of nitrogen on top. Withdraw part of the water, leaving approximately 70% of the batch volume of water in the compounding vessel. Quantitatively add the glycerol with continuous stirring until complete mixing is achieved. Add the active ingredient and the sodium hydroxide solution. Bring the solution to final volume, filter it through a sterilizing membrane and fill into sterile containers with a nitrogen overlay.

| Example 2 | |
|---|---|
| Ingredient | Amount/ml |
| Compound Ia Hydrochloride | 5.51 mg |
| Propylene Glycol | 200 mg |
| Sodium Hydroxide (0.01 N) | 70 μl |
| Water for Injection q.s. ad | 1.00 ml |

Procedure for Formulation

Into a suitable compounding vessel add an excess of the batch volume of Water for Injection. Heat to boiling and allow the water to boil for ten minutes. While cooling, cover the container, bubble nitrogen through the water and keep a jet of nitrogen on top. Withdraw part of the water, leaving approximately 70% of the batch volume of water in the compounding vessel. Quantitatively add the propylene glycol with continuous stirring until complete mixing is achieved. Add the active ingredient and the sodium hydroxide solution. Bring the solution to final volume, filter it through a sterilizing membrane and fill into sterile containers with a nitrogen overlay.

| Example 3 | |
|---|---|
| Ingredient | Amount/ml |
| Compound Ia Hydrochloride | 5.51 mg |
| Ethyl Alcohol | 200 mg |
| Sodium Hydroxide (0.01N) | 55 μ1 |
| Water for injection q.s. ad | 1.00 ml |

Procedure for Formulation

Into a suitable compounding vessel add an excess of the batch volume of Water for Injection. Heat to boiling and allow the water to boil for ten minutes. While cooling, cover the container, bubble nitrogen through the water and keep a jet of nitrogen on top. Withdraw part of the water, leaving approximately 70% of the batch volume of water in the compounding vessel. Quantitatively add the ethyl alcohol with continuous stirring until complete mixing is achieved. Add the active ingredient and the sodium hydroxide solution. Bring the solution to final volume, filter it through a sterilizing membrane and fill into sterile containers with a nitrogen overlay.

| Example 4 | |
|---|---|
| Ingredient | Amount/ml |
| Compound Ia Hydrochloride | 44.1 mg |
| Glycerol | 200 mg |
| Sodium Hydroxide (0.01N) | to pH 6.0-6.2 |
| Water for Injection q.s. ad | 1.00 ml |

Procedure for Formulation

Into a suitable compounding vessel add an excess of the batch volume of Water for Injection. Heat to boiling and allow the water to boil for ten minutes. While cooling, cover the container, bubble nitrogen through the water and keep a jet of nitrogen on top. Withdraw part of the water, leaving approximately 70% of the batch volume of water in the compounding vessel. Quantitatively add the glycerol with continuous stirring until complete mixing is achieved. Add the active ingredient and the sodium hydroxide solution. Bring the solution to final volume, filter it through a sterilizing membrane and fill into sterile containers with a nitrogen overlay.

| Example 5 | |
|---|---|
| Ingredient | Amount/ml |
| Compound Ia Hydrochloride | 11.0 mg |
| Glycerol | 350 mg |
| Sodium Hydroxide (0.01N) | to pH 6.0-6.2 |
| Water for Injection q.s. ad | 1.00 ml |

Procedure for Formulation

Into a suitable compounding vessel add an excess of the batch volume of Water for Injection. Heat to boiling and allow the water to boil for ten minutes. While cooling, cover the container, bubble nitrogen through the water and keep a jet of nitrogen on top. Withdraw part of the water, leaving approximately 70% of the batch volume of water in the compounding vessel. Quantitatively add the glycerol with continuous stirring until complete mixing is achieved. Add the active ingredient and the sodium hydroxide solution. Bring the solution to final volume, filter it through a sterilizing membrane and fill into sterile containers with a nitrogen overlay.

| Example 6 | |
|---|---|
| Ingredient | Amount/ml |
| Compound Ia Hydrochloride | 1.1025 mg |
| Glycerol | 100.0 mg |
| Benzyl Alcohol | 9.0 mg |
| Sodium Hydroxide (1.0N) | to pH 6.3±0.2 |
| Water for Injection q.s. ad | 1.00 ml |

| Example 7 | |
|---|---|
| Ingredient | Amount/ml |
| Compound Ia Hydrochloride | 11.025 mg |
| Glycerol | 100.0 mg |
| Benzyl Alcohol | 9.0 mg |
| Sodium Hydroxide (1.0N) | to pH 6.3±0.2 |
| Water for Injection q.s. ad | 1.00 ml |

| Example 8 | |
|---|---|
| Ingredient | Amount/ml |
| Compound Ia Hydrochloride | 27.56 mg |
| Glycerol | 100.0 mg |
| Benzyl Alcohol | 9.0 mg |
| Sodium Hydroxide (1.0N) | to pH 6.3±0.2 |
| Water for Injection q.s. ad | 1.00 ml |

Procedure for Formulation of Examples 6, 7 and 8

In a suitable vessel, collect 80% of the required amount of Water for Injection and overlay with nitrogen. While stirring, add and dissolve the Glycerol, Benzyl Alcohol and Compound Ia. Agitate to ensure homogeniety and continuously overlay with Nitrogen NF. Adjust the pH of the solution to 6.3 $\pm$ 0.2 by addition of 1N Sodium Hydroxide Solution. Bring the solution to the desired batch volume with Water for Injection, and stir to ensure complete dissolution. Aseptically filter the solution using a sterilized membrane filter, and collect the filtrate in a sterilized receiving vessel. Fill the solution into sterilized ampules, overlay with nitrogen and seal the ampules.

Tables 3 and 4, below, provide stability data for some of the stable injectable formulations of the present invention.

Table 3

| Stability of Compound Ia (5mg/ml) in Ethanol-Water Mixtures at 56° C | | | |
|---|---|---|---|
| Ethanol/Water (v/v) | Calculated Dielectric Constant | % Remaining (4 weeks) | % Remaining (8 weeks) |
| 0/100 | 78.5 | 94.9 | 89.4 |
| 25/75 | 64.9 | 98.7 | 97.5 |
| 50/50 | 51.4 | 99.5 | 99.1 |
| 75/25 | 37.8 | 99.6 | 99.6 |

Table 4

Stability of Compound Ia (5mg/ml) in Propylene Glycol-Water and Glycerol-Water at 56°C

| Solvent | Calculated Dielectric Constant | % Remaining (4 weeks) | %Remaining (8 weeks) |
|---|---|---|---|
| Water | 78.5 | 94.9 | 88.1 |
| 25% (w/v) Propylene Glycol | 67.3 | 97.7 | 96.8 |
| 5% (w/v) Glycerol | 76.3 | 95.1 | 90.9 |
| 15% (w/v) Glycerol | 71.8 | 96.5 | 94.2 |
| 25% (w/v) Glycerol | 71.4 | 96.8 | 95.3 |

**Claims**

**Claims for the following Contracting States : DE, GB, FR, IT, NL, SE, CH/LI, BE, LU**

1. A composition comprising a stable, injectable, aqueous solution of an antiemetic agent of the formula

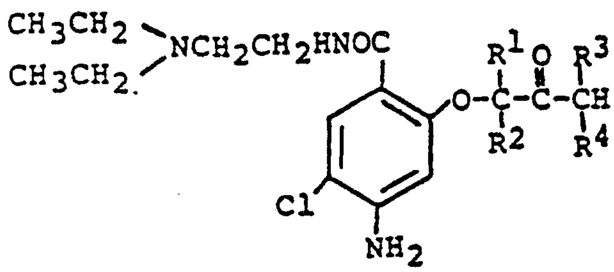

I

wherein $R^1$, $R^2$ and $R^3$ each are independently hydrogen or methyl, and $R^4$ is hydrogen, a straight or branched chain alkyl group containing from 1 to 4 carbon atoms or, when $R^3$ is hydrogen, $R^4$ may be phenyl, or a non-toxic, pharmaceutically acceptable acid addition salt thereof, containing as stabilizing agent a pharmaceutically acceptable, water-miscible, hydroxylic organic solvent or water-soluble polyhydric alcohol or sugar which decreases the dielectric constant of the solution.

2. A composition of Claim 1 wherein the stabilizing agent is selected from ethanol, propylene glycol glycerol and mannitol, and is present in an amount of 5% to 30% (w/v).

3. A composition of Claim 2 wherein the compound of Formula I is present in an amount of 0,1 % to 4% (w/v).

4. A composition of Claim 3 wherein the pH is in the range of 5.0 to 6.7.

5. A composition of Claim 4 wherein the compound of Formula I is 4-amino-2-(2-butanon-3-yl)oxy-5-chloro-N-[2-(diethylamino)ethyl]benzamide.

6. A composition of Claim 5 wherein the stabilizing agent is ethanol, propylene glycol or glycerol.

7. A composition of Claim 6 wherein the compound of Formula I is present as the hydrochloride salt , the stabilizing agent is glycerol and the pH is in the range of 6.0 to 6.5.

8. A composition of Claim 7 wherein the glycerol is present in an amount of about 10% (w/v).

9. A composition of Claim 4 which additionally contains benzyl alcohol as a preservative.

10. A composition of Claim 8 which additionally contains about 9 mg of benzyl alcohol per ml as a preservative.

11. A process for preparing a composition according to anyone of claims 1 to 10 which comprises incorporating as stabilizing agent a pharmaceutically acceptable, water-miscible, hydroxylic organic solvent or water-soluble polyhydric alcohol or sugar which decreases the dielectric constant of the solution into the solution of an antiemetic agent of the formula I as defined in Claim 1.

12. The use of a pharmaceutically acceptable, water-miscible, hydroxylic organic solvent or water-soluble polyhydric alcohol or sugar to decrease the dielectric constant of the solution in a composition comprising an injectable, aqueous solution of an antiemetic agent of the formula I as defined in Claim 1.

**Claims for the following Contracting States : AT, ES, GR**

1. A process for preparing a composition comprising a stable, injectable, aqueous solution of an antiemetic agent of the formula

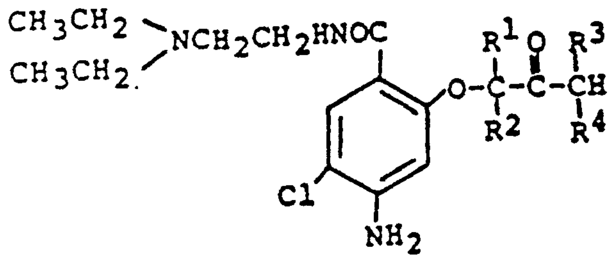

I

wherein $R^1$, $R^2$ and $R^3$ each are independently hydrogen or methyl, and $R^4$ is hydrogen, a straight or branched chain alkyl group containing from 1 to 4 carbon atoms or, when $R^3$ is hydrogen, $R^4$ may be phenyl, or a non-toxic, pharmaceutically acceptable acid addition salt thereof, containing as stabilizing agent a pharmaceutically acceptable, water-miscible, hydroxylic organic solvent or water-soluble polyhydric alcohol or sugar which decreases the dielectric constant of the solution,

which comprises incorporating the pharmaceutically acceptable, water-miscible, hydroxylic organic solvent or water-soluble polyhydric alcohol or sugar which decreases the dielectric constant of the solution into the solution of the antiemetic agent.

2. The process of Claim 1 wherein the stabilizing agent is selected from ethanol, propylene glycol, glycerol and mannitol, and is present in an amount of 5 % to 30 % (w/v).

3. The process of Claim 2 wherein the compound of Formula I is added in such an amount that it is present in the obtained composition in an amount of 0,1 to 4 % (w/v).

4. The process of Claim 3 wherein the pH of the composition obtained is in the range of 5.0 to 6.7.

5. The process of Claim 4 wherein the compound of Formula I is 4-amino-2-(2-butanon-3-yl)oxy-5-chloro-N-[2-(diethylamino)ethyl]benzamide.

6. The process of Claim 5 wherein the stabilizing agent is ethanol, propylene glycol or glycerol.

7. The process of Claim 6 wherein the compound of Formula I is used as the hydrochloride salt, the stabilizing agent is glycerol and the pH of the composition obtained is in the range of 6.0 to 6.5.

8. The process of Claim 7 wherein the glycerol is present in the composition obtained in an amount of about 10 % (w/v).

9. The process of Claim 4 wherein additionally benzyl alcohol is added as a preservative.

10. The process of Claim 8 wherein additionally benzyl alcohol is added in such an amount that the composition obtained contains about 9 mg of benzyl alcohol per ml as a preservative.

11. The use of a pharmaceutically acceptable, water-miscible, hydroxylic organic solvent or water-soluble polyhydric alcohol or sugar for decreasing the dielectric constant of the solution in a composition comprising an injectable, aqueous solution of an antiemetic agent of the formula I as defined in Claim 1.

**Revendications**

**Revendications pour les Etats contractants : DE, GB, FR, IT, NL, SE, CH/LI, BE, LU**

1. Composition caractérisée en ce qu'elle comprend une solution aqueuse stable, injectable, d'un agent antiémétique de la formule

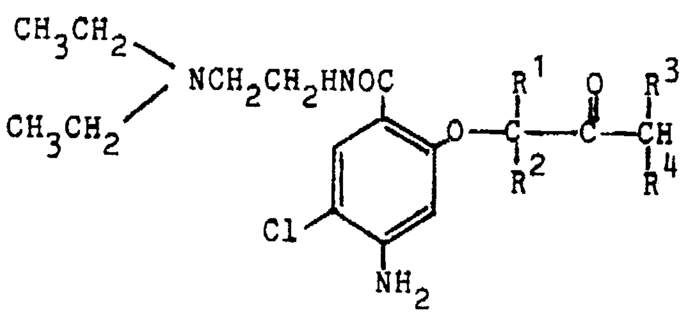

I

où chacun de $R^1$, $R^2$ et $R^3$ est indépendamment hydrogène ou méthyle et $R^4$ est hydrogène, un groupe alkyle à chaîne droite ou ramifiée contenant de 1 à 4 atomes de carbone ou bien lorsque $R^3$ est hydrogène, $R^4$ peut être phényle ou son sel d'addition d'acide non toxique, acceptable en pharmacie, contenant, comme agent stabilisant, un solvant organique hydroxyle acceptable en pharmacie, miscible dans l'eau ou un alcool polyhydrique ou sucre soluble dans l'eau qui diminue la constante diélectrique de la solution.

2. Composition selon la revendication 1, caractérisée en ce que l'agent stabilisant est choisi parmi l'éthanol, le propylène glycol, le glycérol et le mannitol et il est présent en une quantité de 5% à 30% (poids/volume).

3. Composition selon la revendication 2, caractérisée en ce que le composé de formule I est présent en une quantité de 0,1% à 4% (poids/volume).

4. Composition selon la revendication 3, caractérisée en ce que le pH est compris entre 5,0 et 6,7.

5. Composition selon la revendication 4, caractérisée en ce que le composé de formule I est le 4-amino-2-(-2-butanon-3-yl)oxy-5-chloro-N-[2(diéthylamino) éthyl]benzamide.

6. Composition selon la revendication 5, caractérisée en ce que l'agent stabilisant est l'éthanol, le

propylène glycol ou le glycérol.

7. Composition selon la revendication 6, caractérisée en ce que le composé de formule I est présent soins la formule du sel de chlorhydrate, l'agent stabilisant est le glycérol et le pH est compris entre 6,0 et 6,5.

8. Composition selon la revendication 7, caractérisée en ce que le glycérol est présent en une quantité d'environ 10% (poids/volume).

9. Composition selon la revendication 4, caractérisée en ce qu'elle contient additionnellement de l'alcool benzylique comme conservateur.

10. Composition selon la revendication 8, caractérisée en ce qu'elle contient additionnellement environ 9 mg d'alcool benzylique par ml comme conservateur.

11. Procédé de préparation d'une composition selon l'une quelconque des revendications 1 à 10, caractérisé en ce qu'il consiste à incorporer, comme agent stabilisant, un solvant organique hydroxyle acceptable en pharmacie, miscible dans l'eau ou alcool polyhydrique ou sucre soluble dans l'eau qui diminue la constante diélectrique de la solution, dans la solution d'un agent antiémétique de la formule I telle que défini à la revendication 1.

12. Utilisation d'un solvant organique hydroxyle acceptable en pharmacie, miscible dans l'eau ou alcool polyhydrique soluble dans l'eau ou sucre pour diminuer la constante diélectrique de la solution dans une composition comprenant une solution aqueuse injectable d'un agent antiémétique de la formule I selon la revendication 1.

**Revendications pour les Etats contractants suivants : AT, ES, GR**

1. Procédé de préparation d'une composition comprenant une solution aqueuse stable injectable d'un agent antiémétique de la formule

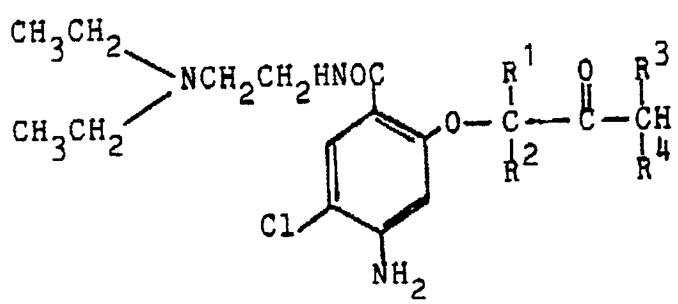

I

où chacun de $R^1$, $R^2$ et $R^3$ est indépendamment hydrogène ou méthyle et $R^4$ est hydrogène, un groupe alkyle à chaîne droite ou ramifiée contenant de 1 à 4 atomes de carbone ou bien lorsque $R^3$ est hydrogène, $R^4$ peut être phényle ou son sel d'addition d'acide non toxique, acceptable en pharmacie, contenant, comme agent stabilisant, un solvant organique hydroxyle acceptable en pharmacie, miscible dans l'eau ou un alcool polyhydrique ou sucre soluble dans l'eau qui diminue la constante diélectrique de la solution,

caractérisé en ce qu'il comprend l'incorporation du solvant organique hydroxyle acceptable en pharmacie, miscible dans l'eau ou alcool polyhydrique ou sucre soluble dans l'eau qui diminue la constante diéléctrique de la solution, dans la solution de l'agent antiémétique.

2. Procédé selon la revendication 1, caractérisé en ce que l'agent stabilisant est choisi parmi l'éthanol, le propylène glycol, le glycérol et le mannitol et est présent une quantité de 5% à 30% (poids/volume).

3. Procédé selon la revendication 2, caractérisé en ce que le composé de formule I est ajouté en une

quantité telle qu'il soit présent dans la composition obtenue en une quantité de 0,1% à 4% (poids/volume).

4. Procédé selon la revendication 3, caractérisé en ce que le pH de la composition obtenue est compris entre 5,0 et 6,7.

5. Procédé selon la revendication 4, caractérisé en ce que le composé de formule I est le 4-amino-2-(2-butanon-3-yl)oxy-5-chloro-N-[2-(diéthylamino)éthyl]benzamide.

6. Procédé selon la revendication 5, caractérisé en ce que l'agent stabilisant est l'éthanol, le propylène glycol, le glycérol.

7. Procédé selon la revendication 6, caractérisé en ce que le composé de formule I est utilisé sous la forme du sel de chlorydrate, l'agent stabilisant est le glycérol et le pH de la composition obtenue est compris entre 6,0 et 6,5.

8. Procédé selon la revendication 7, caractérisé en ce que le glycérol est présent dans la composition obtenue en une quantité d'environ 10% (poids/volume).

9. Procédé selon la revendication 4, caractérisé en ce qu'on ajoute additionnellement de l'alcool benzylique comme conservateur.

10. Procédé selon la revendication 8, caractérisé en ce qu'on ajoute additionnellement de l'alcool benzylique en une quantité telle que la composition obtenue contienne environ 9 mg d'alcool benzylique par ml comme conservateur.

11. Utilisation d'un solvant organique hydroxyle acceptable en pharmacie, miscible dans l'eau ou alcool polyhydrique soluble dans l'eau ou sucre pour diminuer la constante diélectrique de la solution dans une composition comprenant une solution aqueuse injectable d'un agent antiémétique de la formule I selon la revendication 1.

**Patentansprüche**
**Patentansprüche für folgende Vertragsstaaten : DE, GB, FR, IT, NL, SE, CH/LI, BE, LU**

1. Zusammensetzung umfassend eine stabile, injizierbare wässrige Lösung eines antiemetischen Mittels der Formel

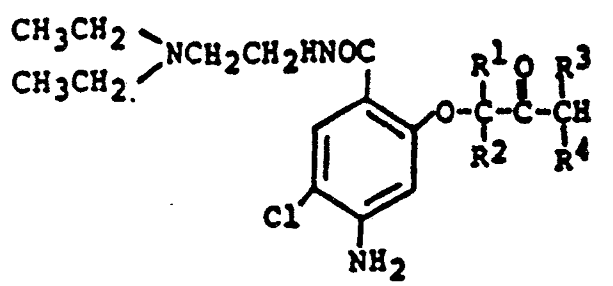

I

wobei $R^1$, $R^2$ und $R^3$ jeweils unabhängig voneinander für Wasserstoff oder Methyl stehen und $R^4$ für ein Wasserstoffatom oder eine geradkettige oder verzweigte Alkylgruppe mit 1 bis 4 Kohlenstoffatomen steht, oder falls $R^3$ für Wasserstoff steht, $R^4$ für Phenyl stehen kann oder ein nicht-toxisches, pharmazeutisch verträgliches Säureadditionssalz davon, die als Stabilisator ein pharmazeutisch verträgliches mit Wasser mischbares organisches, Hydroxygruppen aufweisendes Lösungsmittel oder einen wasserlöslichen Polyalkohol oder Zucker, das oder der die Dielektrizitätskonstante der Lösung verringert, enthält.

2. Zusammensetzung nach Anspruch 1, wobei der Stabilisator ausgewählt ist unter Ethanol, Propylenglycol, Glycerin und Mannit und in einer Menge von 5 bis 30 % (G/V) vorliegt.

3. Zusammensetzung nach Anspruch 2, wobei die Verbindung der Formel I in einer Menge von 0.1 bis 4 % (G/V) vorliegt.

4. Zusammensetzung nach Anspruch 3, wobei der pH-Wert im Bereich von 5.0 bis 6.7 liegt.

5. Zusammensetzung nach Anspruch 4, wobei die Verbindung der Formel I 4-Amino-2-(2-butanon-3-yl)-oxy-5-chlor-N-(2-(diethylamino)ethyl)benzamid ist.

6. Zusammensetzung nach Anspruch 5, wobei der Stabilisator Ethanol, Propylenglycol oder Glycerin ist.

7. Zusammensetzung nach Anspruch 6, wobei die Verbindung der Formel I in Form des Hydrochloridsalzes vorliegt, der Stabilisator Glycerin ist und der pH-Wert im Bereich von 6.0 bis 6.5 liegt.

8. Zusammensetzung nach Anspruch 7, wobei das Glycerin in einer Menge von etwa 10 % (G/V) vorliegt.

9. Zusammensetzung nach Anspruch 4, welche außerdem Benzylalkohol als Konservierungsmittel enthält.

10. Zusammensetzung nach Anspruch 8, welche außerdem etwa 9 mg Benzylalkohol pro ml als Konservierungsmittel enthält.

11. Verfahren zur Herstellung einer Zusammensetzung nach einem der Ansprüche 1 bis 10, wobei man der Lösung eines antiemetischen Mittels der Formel I wie in Anspruch 1 definiert, ein pharmazeutisch verträgliches, mit Wasser mischbares, organisches, Hydroxygruppen aufweisendes Lösungsmittel oder einen Polyalkohol oder Zucker, das oder der die dielektrische Konstante der Lösung verringert, als Stabilisator einverleibt.

12. Verwendung eines pharmazeutisch verträglichen, mit Wasser mischbaren, organischen, Hydroxygruppen aufweisendes Lösungsmittels oder eines wasserlöslichen Polyalkohols oder Zuckers zur Verringerung der Dielektrizitätskonstante der Lösung in einer Zusammensetzung, umfassend eine injizierbare, wässrige Lösung eines antiemetischen Mittels der Formel I wie in Anspruch 1 definiert.

**Patentansprüche für folgende Vertragsstaaten : AT, ES, GR**

1. Verfahren zur Herstellung einer Zusammensetzung umfassend eine stabile, injizierbare wässrige Lösung eines antiemetischen Mittels der Formel

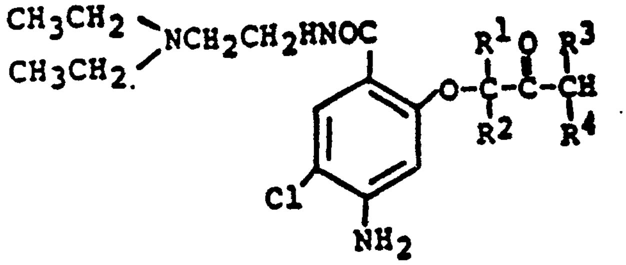

I

wobei $R^1$, $R^2$ und $R^3$ jeweils unabhängig voneinander für Wasserstoff oder Methyl stehen und $R^4$ für ein Wasserstoffatom oder eine geradkettige oder verzweigte Alkylgruppe mit 1 bis 4 Kohlenstoffatomen steht, oder falls $R^3$ für Wasserstoff steht, $R^4$ für Phenyl stehen kann oder ein nicht-toxisches, pharmazeutisch verträgliches Säureadditionssalz davon, die als Stabilisator ein pharmazeutisch verträgliches mit Wasser mischbares, organisches, Hydroxygruppen aufweisendes Lösungsmittel oder einen wasserlöslichen Polyalkohol oder Zucker, das oder der die Dielektrizitätskonstante der Lösung verringert, enthält,
wobei man das pharmazeutisch verträgliche, mit Wasser mischbare, organische, Hydroxygruppen aufweisende Lösungsmittel oder einen wasserlöslichen Polyalkohol oder Zucker, das oder der die Dielektrizitätskonstante der Lösung verringert, der Lösung des antiemetischen Mittels einverleibt.

2. Verfahren nach Anspruch 1, wobei der Stabilisator ausgewählt ist unter Ethanol, Propylenglycol, Glycerin und Mannit und in einer Menge von 5 bis 30 % (G/V) vorliegt.

3. Verfahren nach Anspruch 2, wobei die Verbindung der Formel I in einer Menge von 0.1 bis 4 % (G/V) vorliegt.

4. Verfahren nach Anspruch 3, wobei der pH-Wert der erhaltenen Zusammensetzung im Bereich von 5.0 bis 6.7 liegt.

5. Verfahren nach Anspruch 4, wobei die Verbindung der Formel I 4-Amino-2-(2-butanon-3-yl)oxy-5-chlor-N-(2-(diethylamino)-ethyl)benzamid ist.

6. Verfahren nach Anspruch 5, wobei der Stabilisator Ethanol, Propylenglycol oder Glycerin ist.

7. Verfahren nach Anspruch 6, wobei die Verbindung der Formel I als Hydrochloridsalz verwendet wird, der Stabilisator Glycerin ist und der pH-Wert der erhaltenen Zusammensetzung im Bereich von 6.0 bis 6.5 liegt.

8. Verfahren nach Anspruch 7, wobei das Glycerin in der erhaltenen Zusammensetzung in einer Menge von etwa 10 % (G/V) vorliegt.

9. Verfahren nach Anspruch 4, wobei außerdem Benzylalkohol als Konservierungsmittel hinzugefügt wird.

10. Verfahren nach Anspruch 8, wobei außerdem Benzylalkohol in einer Menge hinzugefügt wird, daß die erhaltene Zusammensetzung etwa 9 mg Benzylalkohol pro ml als Konservierungsmittel enthält.

11. Verwendung eines pharmazeutisch verträglichen, mit Wasser mischbaren, organischen, Hydroxygruppen aufweisenden Lösungsmittels oder eines wasserlöslichen Polyalkohols oder Zuckers zur Verringerung der Dielektrizitätskonstante der Lösung in einer Zusammensetzung, umfassend eine injizierbare, wässrige Lösung eines antiemetischen Mittels der Formel I wie in Anspruch 1 definiert.